(19) Europäisches Patentamt
European Patent Office
Office européen des brevets

(11) Publication number: **0 205 025**
**A1**

# (12) EUROPEAN PATENT APPLICATION

(21) Application number: 86107131.4

(22) Date of filing: 26.05.86

(51) Int. Cl.[4]: C 07 C 172/00
A 61 K 31/59, C 07 J 9/00

(30) Priority: 27.05.85 JP 113866/85
31.03.86 JP 73825/86

(43) Date of publication of application:
17.12.86 Bulletin 86/51

(84) Designated Contracting States:
AT BE CH DE FR GB IT LI NL SE

(71) Applicant: SUMITOMO PHARMACEUTICALS COMPANY, LIMITED
40, Dosho-machi 2-chome
Higashi-ku Osaka-shi Osaka-fu(JP)

(71) Applicant: Morii, Hirotoshi
8-57, Koyodai 3-chome
Kawanishi-shi(JP)

(72) Inventor: Morii, Hirotoshi
8-57, Koyoda-3-chome
Kawanishi-shi(JP)

(72) Inventor: Hamma, Noritaka
140-15, Hikisho-Haraderamchi
Sakai-shi(JP)

(72) Inventor: Saito, Yoshikazu
4-1-104, Ryodocho
Nishinomiya-shi(JP)

(72) Inventor: Nishizawa, Toshio
27-E-501, Shinashiyakami
Suita-shi(JP)

(72) Inventor: Nagata, Akihiko
3-2-102, Ichigayacho
Nishinomiya-shi(JP)

(74) Representative: VOSSIUS & PARTNER
Siebertstrasse 4 P.O. Box 86 07 67
D-8000 München 86(DE)

## (54) Fluorine derivatives of vitamin D3 and cell differentiation-inducing agents containing the same as an active ingredient.

(57) Described are novel compounds of the formula (IV)

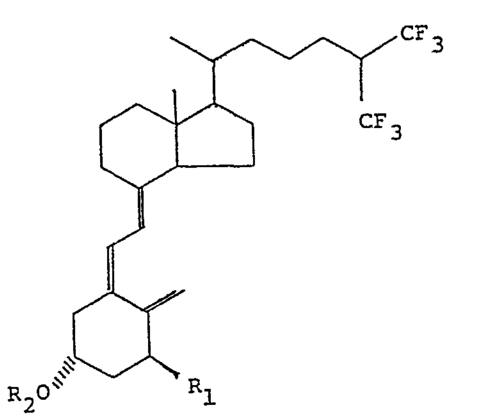

[IV]

wherein $R_1$ is a hydrogen atom, a hydroxyl group or a protected hydroxyl group and $R_2$ is a hydrogen atom or a protecting group,

a pharmaceutical composition useful as a cell differentiation-inducing agent, which contains an effective amount of a compound of the formula IV (wherein $R_2$ is H) as an active ingredient and a pharmaceutically acceptable carrier or diluent,

a method of inducing cell differentiation by administering an effective amount of a compound of the formula IV (wherein $R_2$ is H) to a patient,

and a process for producing a compound of the formula (IV) by subjecting a previtamin $D_3$ derivative of the formula (III)

[III]

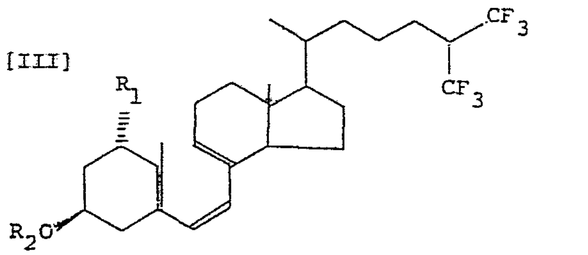

wherein $R_1$ and $R_2$ are as defined above, to thermal isomerization to yield a vitamin $D_3$ derivative of the formula (IV) mentioned above.



Croydon Printing Company Ltd.

# FLOURINE DERIVATIVES OF VITAMIN $D_3$ AND CELL DIFFERENTIATION-INDUCING AGENTS CONTAINING THE SAME AS AN ACTIVE INGREDIENT

## TECHNICAL FIELD

This invention relates to a novel fluorine derivative of vitamin $D_3$. More particularly, it relates to a novel fluorine derivative of vitamin $D_3$ which not only has an excellent pharmacological activity, namely a useful vitamin D- like physiological activity, and is useful as a curative or preventive medicine for various diseases caused by disorders of transportation, absorption or metabolism of calcium, for example bone diseases such as rickets, osteomalacia and osteoporosis, but also has an ability to suppress the proliferation of tumor cells such as myeloleukemia cells and to induce the differentiation of these cells into normal cells, thus is useful as an antitumor agent, and further has an antirheumatic activity.

## PRIOR ART

It is known that 1α, 25-dihydroxyvitamim $D_3$ which is a metabolite of vitamin $D_3$ in a living body and is known as the active-form of vitamin $D_3$, and its artificial homologues, 1α-hydroxyvitamin $D_3$, 1α, 24-dihydroxyvitamim $D_3$ and the like, exhibit an

action of stimulating the absorption of calcium from the intestine and are effective as curatives for bone diseases and the like. Further, there has been found recently in vitamin $D_3$ and its analogous compounds a differentiation-inducing action to restore cancerated cells into normal cells. Actually, some of these compounds have been found to have an antitumor activity (Y. Honma et al., Proc. Natl. Acad. Sci., USA, 80, 201 (1983)), and is attracting attention. However, the results obtained so far are still unsatisfactory.

On the other hand, among the other homologues of 26,26,26,27,27,27-hexafluorovitamin $D_3$ and its derivatives which are the objective compounds of this invention, 26,26,26,27,27,27-hexafluoro-25-hydroxyvitamin $D_3$ (U.S. Patent No. 4,248,791) and 26,26,26,27,27,27-hexafluoro-1α, 25-dihydroxyvitamin $D_3$ (U.S. Patent No. 4,358,406) are known to have a high, vitamim D-like physiological activity, and their effectiveness as an antitumor agent is disclosed in Japanese Patent Application Kokai (Laid-open) No. 7215/86.

However, the vitamim D analogues of the above-mentioned kind have, when used in medical treatment as antitumor agents or antirheumatic agents, a problem of side effect caused by their inherent vitamin D action, such as hypercalcemia, and hence are not yet used at present as practically useful curative agents.

OBJECT OF THE INVENTION

The object of this invention is to provide a 26,26,26,27,27,27-hexafluorovitamin $D_3$ derivative which is a novel compound and has an excellent pharmacological activity.

More particularly, the object of this invention is to provide a 26,26,26,27,27,27-hexafluorovitamin $D_3$ derivative which not only exhibits a useful vitamim D-like physiological action, namely an action of stimulating the absorption of calcium from the intestine and the like action, and is useful as a curative agents for bone diseases and the like, but also, even in a low dosage in which the above-mentioned vitamim D-like physiological activity is not manifested, shows a powerful cell differentiation-inducing action and is useful in the treatment of leukemia and the like as an antitumor agent having a low degree of influence on the metabolism of calcium in a living body and a low toxicity, and further has an excellent antirheumatic activity.

CONSTRUCTION AND EFFECT OF THE INVENTION

The hexafluorovitamin $D_3$ derivative provided according to this invention is represented by the formula [IV]

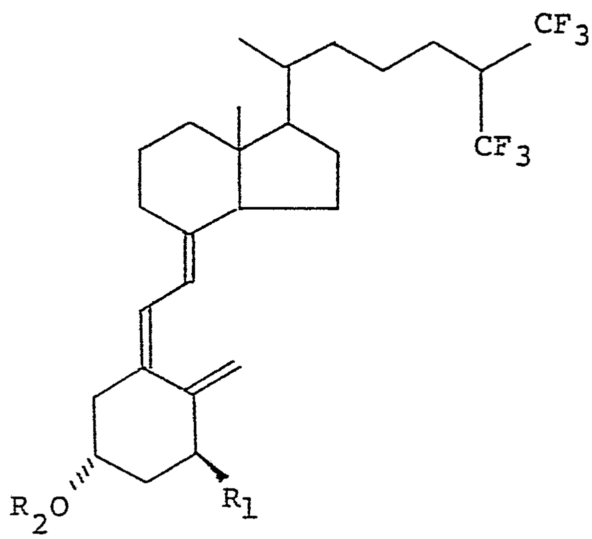

[IV]

wherein $R_1$ is a hydrogen atom, a hydroxyl group or a protected hydroxyl group and $R_2$ is a hydrogen atom or a protecting group. The compound wherein, in the above-mentioned formula [IV], $R_1$ is a hydrogen atom or a hydroxyl group and $R_2$ is a hydrogen atom, namely the compound represented by the formula [I]

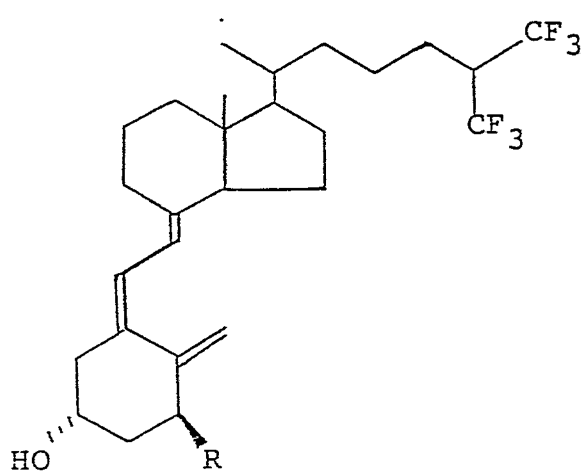

[I]

0205025

wherein R is a hydrogen atom or a hydroxyl group, exhibits a vitamin D- like action or a cell differentiation-inducing action, and hence is useful as a curative or preventive medicine for bone diseases, or as a cell differentiation-inducing agent or an antitumor agent. As specific examples of the compound represented by the formula [I], mention may be made of 26,26,26-hexafluoro-1α-hydroxyvitamin $D_3$ (compound A) and 26,26,26-hexafluorovitamin $D_3$ (compound B).

Further, the compound wherein, in the above-mentioned formula [IV], $R_1$ is a protected hydroxyl group or $R_2$ is a protecting group, is useful as an intermediate for producing the compound represented by the above-mentioned formula [I].

The above-mentioned compound of the formula [IV] according to this invention can be prepared by various methods known to the art as the method of preparing vitamin $D_3$ and its analogues. A typical example of the methods will be described below.

A cholesta-5,7-diene represented by the formula [II]

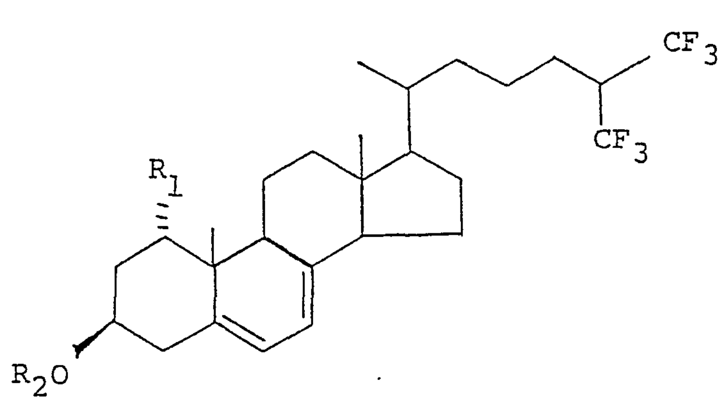

[II]

wherein $R_1$ and $R_2$ are as defined above, is irradiated with ultraviolet light, and the resutling previtamin $D_3$ derivative represented by the formula [III]

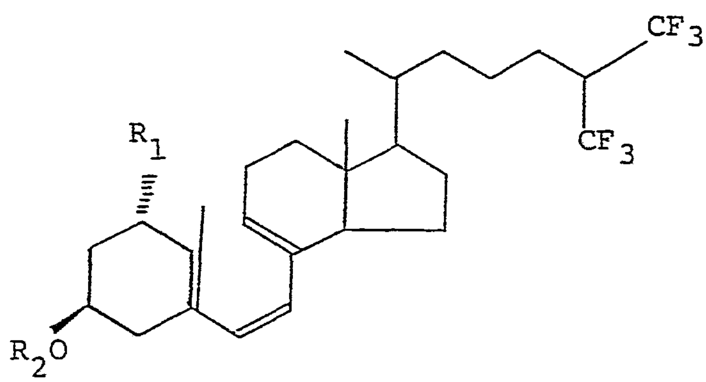

[III]

wherein $R_1$ and $R_2$ are as defined above, is thermally isomerized to prepare the compound of this invention represented by the above-mentioned formula [IV]. Further, the compound wherein, in the above-mentioned formula [III], $R_1$ is a protected hydroxyl group or $R_2$ is a protecting group, can be converted to the above-mentioned compound of the formula [I] by subjecting the former to deprotection reaction.

The protecting group referred to in this invention for $R_1$ or $R_2$ is used for the purpose of protecting a hydroxyl group in the above-mentioned ultraviolet irradiation or thermal isomerization. Such protecting groups are not restricted specifically so long as they can achieve the said purpose. As typical examples thereof, mention may be made of acyl groups

such as a formyl group, an alkanoyl group and an aromatic acyl group, alkyloxycarbonyl groups, and aralkyloxycarbonyl groups. Examples of alkanoyl groups include alkanoyl groups having 2 to 6 carbon atoms such as acetyl, chloroacetyl, propionyl and pivaloyl; those of aromatic acyl groups include benzoyl groups optionally substituted with a nitro group, a halogen atom, an alkoxy group having 1 to 4 carbon atoms, and an alkyl group having 1 to 4 carbon atoms, such as benzoyl, p-nitrobenzoyl, o-nitrobenzoyl, p-chlorobenzoyl, o-chlorobenzoyl, p-methoxybenzoyl, o-methoxybenzoyl, and p-methylbenzoyl; those of alkyloxycarbonyl groups include alkyloxycarbonyl groups having an alkyl group of 1 to 4 carbon atoms, such as ethoxycarbonyl; those of aralkyloxycarbonyl groups include, for example, benzyloxycarbonyl groups whose phenyl group may be optionally substituted with a nitro group or a halogen atom, such as benzyl-, p-nitrobenzyl-, o-nitrobenzyl-, p-chlorobenzyl- and o-chlorobenzyl-oxycarbonyl. Particularly preferred examples of protecting groups include an acetyl group and a benzoyl group.

In the above-mentioned method, the step of ultraviolet irradiation is carried out by irradiating a compound represented by the general formula (II) with ultraviolet light in a suitable inert solvent, for example organic solvents such as benzene, toluene, methanol, ethanol, n-hexane and diethyl ether or a

mixture thereof and in an atmosphere of inert gas such as nitrogen and argon. The source of ultraviolet light may be those conventionally used, including, for example, a mercury lamp as an easily available one. A filter may be used together according to necessity. An irradiation temperature of -10 to 40°C, preferably -10 to 20°C, gives good results. Although the irradiation time varies depending on the kind of ultraviolet source, the concentration of the starting compound of the formula [II] and the kind of solvents, it is usually several to several tens of minutes.

The previtamin $D_3$ derivative represented by the general formula [III] formed by the above-mentioned ultraviolet irradiation may be isolated by subjecting a crude product obtained after distilling off the solvent from the reaction mixture to a conventional separation procedure such as chromatography. However, it is also possible to carry out thermal isomerization by heating the reaction mixture without isolating the previtamin $D_3$ derivative of the formula [III] after the completion of the ultraviolet-irradiated reaction. Thus, thermal isomerization is conducted by heating the reaction mixture, after the irradiation of ultraviolet light, at 20 to 120°C, preferably 50 to 100°C, for about 2 to 5 hours. This reaction is preferably carried out in an inert gas such as nitrogen and argon. The isolation of the compound of the formula [IV] from the reaction

mixture is effected, after the solvent has been evaporated off, by conventional methods of separation such as chromatography.

When the compound of the formula [IV] thus obtained is in a protected form, namely $R_1$ is a protected hydroxyl group or $R_2$ is a protecting group, it may be subjected to a deprotection reaction to be converted into the 26,26,26,27,27,27-hexafluorovitamin $D_3$ derivative having the formula [I]. The deprotection of the hydroxyl group may be effected by using a reaction known per se. For example, the compound in a protected form may be treated in an alkaline solution of a lower aliphatic alcohol such as methanol or ethanol, or treated in a solvent such as ether or tetrahydrofuran with a metal hydride complex such as lithium aluminum hydride. The reaction temperature for these deacylations may be -20 to 50°C in every case. Usually, room temperature is satisfactorily used.

Thus, the intended compound of the formula [I] of this invention is obtained.

The starting compound of the formula [II] may be prepared by using a prior art process known as the method of preparing cholesta-5,7-dienes, but it can advantageously prepared according to the method described below, for example. As a specific example, a method of preparation will be illustrated below for a compound of the formula [IIa], a compound wherein $R_1$ and

0205025

$R_2$ in the general formula [I] are an acetoxy group (OAc) and an acetyl group (Ac), respectively, and for a compound of the formula [IIb], a compound wherein $R_1$ and $R_2$ in the said formula [I] are a hydroxyl group and a hydrogen atom, respectively.

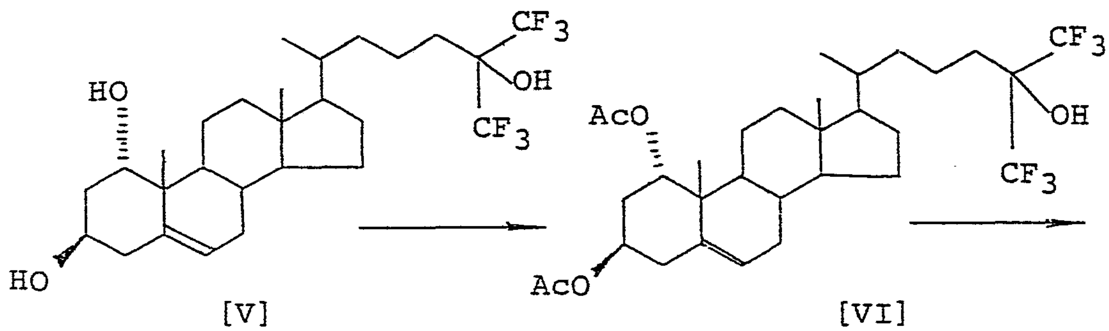

[V] [VI]

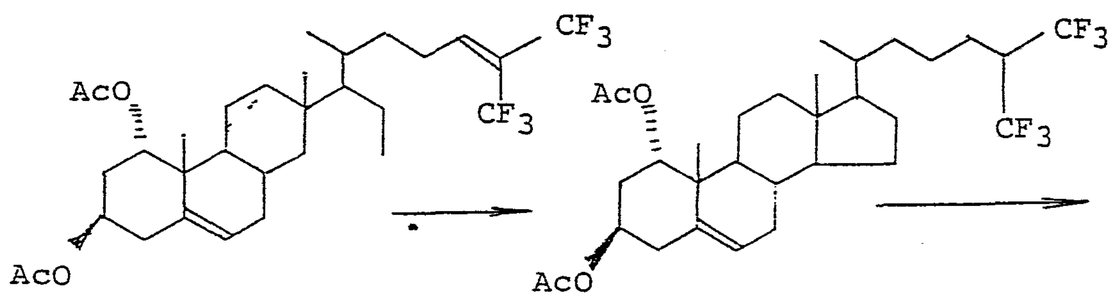

[VII] [VIII]

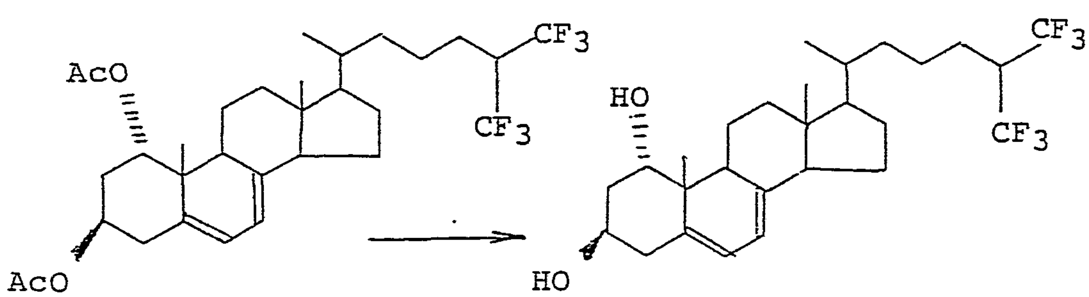

[IIa] [IIb]

First, 1α, 25-dihydroxy-26,26,26,27,27,27-hexafluorocholesterol [V] obtained by the method described in U.S. Patent No. 4,358,406 or Japanese National Publication (Kohyo) No. 500864/84 is treated by a method known per se, for example treated with acetic anhydride in pyridine, to obtain its 1,3-diacetate [VI]. The dehydration of the compound [VI] proceeds in approximately quantitative yield when it is heated together with triphenylphosphine and carbon tetrachloride in a suitable inert solvent at 50 to 100°C, to give the compound [VII]. Then, the compound [VII] is hydrogenated by the use of conventional hydrogenation catalysts such as palladium to give the compound [VIII]. The latter is then brominated by a known method, for example with N-bromosuccinic imide or 1,3-dibromo-5,5-dimethylhydantoin, and then subjected to dehydrobromination by using a base such as s-collidine, to give the 5,7-diene compound [IIa]. The compound [IIa] thus obtained can be converted as desired into the compound [IIb] by deacetylation according to a method known per se, for example hydrolysis under alkaline conditions or hydrogenation with a metal hydride complex such as lithium aluminum hydride.

The compound wherein $R_1$ in the general formula [II] is a hydrogen atom can also be prepared similarly. Namely, the compound represented by the general formula [II] wherein the 1α-position is occupied by a hydrogen

atom can be obtained by using 26,26,26,27,27,27-hexafluoro-25-hydroxycholesterol in place of the above-mentioned starting material represented by the formula [V] and treating it through substantially the same reaction route as that described above.

The compound [I] thus obtained is administered either parenterally, for example injected intramuscularly, subcutaneously or intravenously or applied as ointment, or orally. The dosage for adult per one day is 1 to 2000 μg, preferably 50 to 1000 μg, in the treatment of bone diseases, and 0.1 to 200 μg, preferably 0.1 to 50 μg, as a differentiation-inducing agent, and is increased or decreased appropriately in accordance with the strength of pharmacological response.

The pharmaceutical preparations of the compound [I] are prepared in combination thereof with pharmaceutically acceptable carriers known to the art, which carriers may be either solid or liquid. Specific examples of carriers to be used include maize starch, olive oil, sesame oil, and a trigluceride of medium-chain fatty acid generally called MCT. The dosage forms used include tablets, capsels, liquids, powders, and granules.

This invention will be described further in detail below with reference to Examples. The compound numbers assigned to each compound in the above description are used without change also in the Examples.

Example 1

Preparation of 26,26,26,27,27,27-hexafluoro-1α-hydroxyvitamin $D_3$ (compound A)

(1) Preparation of compound [VI]

A mixture of 2.5 g of 26,26,26,27,.27,27-hexafluoro-1α, 3α, 25-trihydroxycholest-5-en [V] obtained by the method described in Japanese National Publication (Kohyo) No. 501176/83, 30 ml of pyridine, and 5 g of acetic anhydride was stirred at room temperature for 18 hours. A 200 ml volume of ice water was added to the reaction mixture, and the resulting mixture was extracted with toluene. The toluene layer was washed successively with 1N-HCl, aqueous sodium bicarbonate solution and water, and then toluene was distilled off under vacuum. The residue was dissolved in 50 ml of methanol, mixed with 5 ml of 28% aqueous ammonia, and allowed to stand at room temperature for 1 hour. Then, 300 ml of aqueous sodium chloride solution and 100 ml of toluene were added to the solution, and the mixture was subjected to extraction. The toluene layer was washed with water, and concentrated under vacuum. The residue was mixed with n-hexane and concentrated again. The resulting residue was dried under vacuum (1 mmHg) to obtain 2.35 g of the compound [VI] as a white amorphous powder.

IR ($CHCl_3$, $cm^{-1}$): 3350, 1720

NMR ($CDCl_3$, δ):

0.67 (3H, s), 0.92 (3H, d, J=6.6Hz), 1.08 (3H, s), 2.02 (3H, s), 2.05 (3H, s), 2.85 (1H, s), 4.92 (1H, m), 5.06 (1H, b-s), 5.53 (1H, m)

(2) Preparation of compound [VII]

In 50 ml of 1,2-dichloroethane, were dissolved 2 g of the compound [VI], 2.6 g of triphenylphosphine, and 2 ml of carbon tetrachloride. The mixed solution was refluxed for 20 minutes, then the solvent was distilled off under vacuum. The residue was purified by silica gel column chromatography (solvent system: ethyl aceate-n-hexane 1:9), and crystallized from methanol to give 1.86 g of the compound [VII] showing a melting point of 96 to 97°C.

IR (Nujol, $cm^{-1}$): 1740, 1735, 1670

NMR ($CDCl_3$, δ):

0.68 (3H, s), 0.95 (3H, d, J=6.6Hz), 1.08 (3H, x), 2.02 (3H, s), 2.05 (3H, s), 4.92 (1H, m), 5.06 (1H, b-s), 5.52 (1H, m), 6.72 (1H, t, J=7.7Hz)

(3) Preparation of compound [VIII]

One gram of the compound [VII] obtained in (2) above was dissolved in 100 ml of ethanol, and 0.1 g of 5% Pd-C was added to the solution. The resulting mixture was stirred at room temperature in hydrogen atmosphere. When the volume of absorbed gas reached 40 ml,

the reaction mixture was filtered, and the filtrate was concentrated under vacuum to obtain 0.98 g of the compound [VIII] in a powder form. When crystallized in methanol, the product showed a melting point of 86 to 88°C.

IR (Nujol, $cm^{-1}$): 1740 (ester)

NMR ($CDCl_3$, $\delta$):

0.67 (3H, s), 0.93 (3H, d, J=6.3Hz), 1.08 (3H, s), 2.02 (3H, s), 2.05 (3H, s), 2.82 (1H, m), 4.92 (1H, m), 5.06 (1H, m), 5.53 (1H, m)

(4) Preparation of compound [IIa]

A solution of 592 mg of the compound [VIII] in 20 ml of carbon tetrachloride was heated under reflux in a nitrogen gas stream, and 214 mg of N-bromosuccinic imide was added thereto. The reaction liquid was refluxed for 20 minutes, then cooled with ice, the precipitate was removed by filtration, and the filtrate was concentrated under vacuum. The residue was dissolved in 5 ml of xylene, and the solution was added dropwise to a refluxing solution consisting of 20 ml of xylene and 7 ml of 2,4,6-collidine over a period of 3 minutes. Then, the resulting mixture was refluxed in nitrogen atmosphere for 30 minutes. The reaction mixture was cooled down to room temperature, then 50 ml of ethyl acetate was added thereto, and the resulting mixture was washed successively with 2N hydrochloric acid, 5% aqueous sodium bicarbonate solution and water,

and concentrated under vacuum. The residue was dissolved in 40 ml of acetone, then mixed with 0.1 g of p-toluenesulfonic acid, and allowed to stand in the dark in a nitrogen atmosphere at room temperature for 20 hours. The reaction mixture was concentrated under vacuum and purified by silica gel column chromatography (solvent system: ethyl acetate-n-hexane 1:15) to obtain 112 mg of the compound [IIa].

uv spectrum (ethanol, nm): $\lambda_{max}$

293, 281, 271, 262 (shoulder)

NMR ($CDCl_3$, $\delta$):

0.62 (3H, s), 0.94 (3H, d, J=4.3Hz), 1.01 (3H, s), 2.08 (3H, s), 2.03 (3H, s), 2.83 (1H, m), 4.99 (2H, b-s), 5.39 (1H, b-s), 5.67 (1H, b-s)

(5) Preparation of compound A

A 22 mg portion of the compound [IIa] was dissolved in 330 ml of deoxygenated benzene-ethanol mixture (2:1). The solution was cooled to 2 to 5°C, and irradiated with ultraviolet light for 20 minutes by means of a 160W low pressure mercury lamp while argon gas was bubbled through the solution. The solution was then heated under reflux in argon gas for 3 hours, and thereafter concentrated under vacuum. The crude 1α-acetoxy-26,26,26,27,27,27-hexafluorovitamin $D_3$ acetate ([IV], $R_1$ = acetoxy) thus obtained was dissolved in 10 ml of 5% KOH-methanol and allowed to stand in argon gas in the dark for 5 hours. The reaction mixture was

acidified by the addition of 2N HCl, extracted with ethyl acetate, and the ethyl acetate layer was washed with water and then concentrated under vacuum at a temperature of 40°C or less. The crude product obtained was purified twice by silica gel column chromatography (solvent system: ethyl acetate-n-hexane 3:7 and dichloromethane-methanol 50:1) to obtain 3.1 mg of intended 26,26,26,27,27,27-hexafluoro-1α-hydroxyvitamin $D_3$ (compound A).

uv (ethanol, nm): $\lambda_{max}$ 264.5, $\lambda_{min}$ 227

NMR ($CDCl_3$, δ):

0.55 (3H, s), 0.93 (3H, d, J=6.3Hz), 2.82 (1H, m), 4.23 (1H, m), 4.43 (1H, m), 5.00 (1H, s), 5.33 (1H, s), 6.02 (1H, d, J=11.3 Hz) 6.38 (1H, d, J=11.3Hz)

Example 2

Preparation of 26,26,26,27,27,27-hexafluoro-1α-hydroxyvitamin $D_3$ (compound A)

(1) Preparation of compound [IIb]

A 38 mg portion of 1α, 3α-diacetoxy-26,26,26,27,27,27-hexafluorocholesta-5,7-diene [IIa] obtained in Example 1 was dissolved in 3 ml of 5% NaOH-methanol, and allowed to stand at room temperature for 5 hours. To the resulting reaction mixture were added 30 ml of water and 20 ml of ether, and the mixture was extracted. The ether layer was washed with water, dried

over $MgSO_4$, and the solvent was distilled off under vacuum to obtain 31 mg of the compound [IIb].

uv spectrum ethanol, nm): $_{max}$ 293, 281, 271

NMR ($CDCl_3$, $\delta$):

0.63 (3H, s), 0.94 (3H, s), 0.96 (3H, d, J=6.6Hz), 2.84 (1H, m), 3.75 (1H, b-s), 4.05 (1H, m), 5.37 (1H, m) 5.77 (1H, m)

(2) Preparation of compound A

A 10 mg portion of the compound [IIb] was dissolved in 200 ml of deoxygenated benzene-ethanol mixture (2:1). The solution was cooled to 0 to 5°C, and irradiated for 10 minutes by means of a 160W low pressure mercury lamp while argon gas was bubbled through the solution. The solution was then heated under reflux in argon atmosphere for 3 hours, and the resulting reaction mixture was concentrated under vacuum. The crude product of the residue was purified twice by column chromatography using 30 g of silica gel (solvent system: ethyl acetate-n-hexane 3:7 and dichloromethane-methanol 50:1) to obtain the intended compound A. This product showed substantially the same UV and NMR spectrum as those obtained in in Example 1.

Example 3

Differentiation of human premyeloblast leukemia cells (HL-60) into macropharges induced by compound A

Experimental methods

Proliferation-suppression rate

An HL-60 cell fluid adjusted to a concentration of 5 x $10^4$ cells/ml was incorporated with each of the agents to be tested and cultivated in a carbon dioxide incubator at 37°C for 4 days. After the cultivation, the number of cells was measured by means of a Coulter counter. The percentage of the number thus measured relative to the number of cells in an untreated group was calculated, from which the proliferation-suppression rate was obtained.

NBT reduction

HL-cells were treated with the agent to be tested for 4 days, and then a growth medium (95% RPMI-1640, 5% FCS) and a 0.2% 12-0-tetradecanoylphorbol-13-acetate (TPA, 200 ng/ml) solution were added thereto in an equal amount, and the resulting mixture was cultivated at 37°C for 30 minutes. Thereafter, the cells were smeared onto a slide glass, subjected to giemsa staining, and the coloration of cells was examined under a microscope. The number of colored cells was measured for 200 cells, and the results were expressed in terms of the percentage of NBT reduction-

1 positive cells.

Binding property of monoclonal antibody

HL-60 cells wre treated with the agent as described above, and then stained by indirect fluorescent antibody technique using monoclonal antibodies [MAS 072 (Sera-lab) anti-human monocyte and OKT 9 (Ortho-mune) anti-human transferrin receptor] and a fluorescent antibody [FITC-anti-mouse IgG (Miles)], and the resulting fluorescence intensity was determined by FACS to obtain the binding property of the monoclonal antibody in terms of a percentage.

Results of experiments

The results obtained are shown in Table 1. Table 1 reveals clearly that HL-60 cells have been induced to differentiate into macrophages by treatment with the compound of this invention.

Table 1 Morphological and functional changes of HL-60 cells after differentiation induction

| Agent and concentration (M) | Compound A | | $1\alpha$, 25-$(OH)_2D_3$*) | | Control |
|---|---|---|---|---|---|
| | $1x10^{-8}$ | $3x10^{-9}$ | $1x10^{8}$ | $3x10^{-9}$ | |
| Proliferation suppression rate (%) | 74.9 | 58.5 | 66.0 | 47.2 | 0 |
| NBT reduction rate (%) | 91.5 | 77.0 | 87.0 | 48.5 | 0 |
| Binding property of monoclonal antibody MAS072 (%) | 79.0 | | 55.8 | | 17.0 |
| Binding property of monoclonal antibody OKT9 (%) | 11.1 | 16.7 | 22.6 | 61.5 | 95.7 |

Note: *) $1\alpha$, 25-dihydroxyvitamin $D_3$

We claim:

1. A compound of the formula [IV]

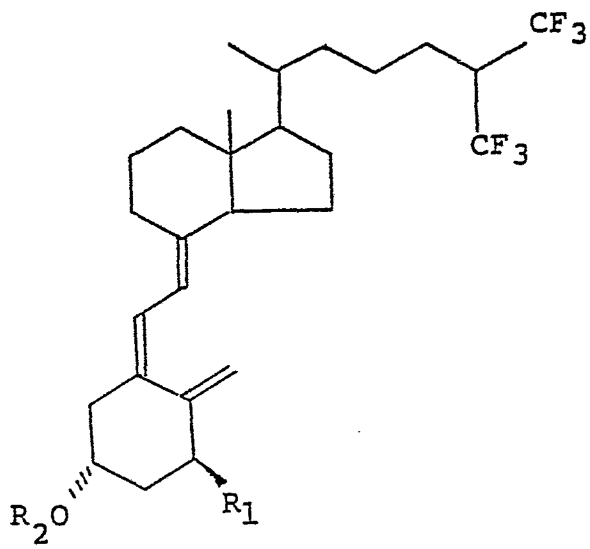

[IV]

wherein $R_1$ is a hydrogen atom, a hydroxyl group or a protected hydroxyl group and $R_2$ is a hydrogen atom or a protecting group.

2. A compound as claimed in Claim 1 which is represented by the formula [I]

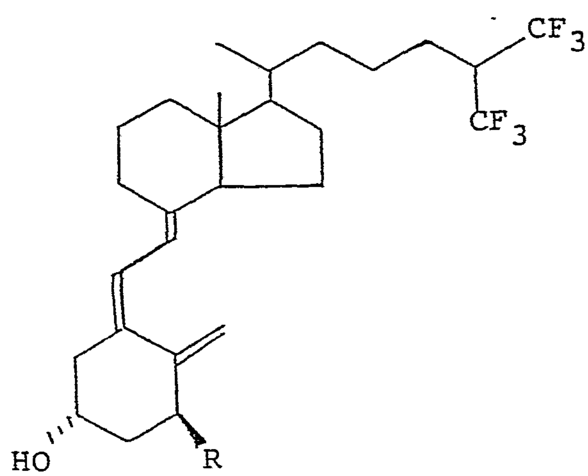

[I]

wherein R is a hydrogen atom or a hydroxyl group.

3. 26,26,26,27,27,27-Hexafluoro-1α-hydroxyvitamin $D_3$.

4. 26,26,26,27,27,27-Hexafluorovitamin $D_3$.

5. A pharmaceutical composition useful as a cell differentiation-inducing agent, which comprises an effective amount of a compound of Claim 2 as an active ingredient and a pharmaceutically acceptable carrier or diluent.

6. A method of inducing cell differentiation which comprises administering an effective amount of a compound of Claim 2 to a patient.

7. A compound of Claim 2 for use as an active therapeutic substance.

8. A process for producing a compound of the formula [IV]

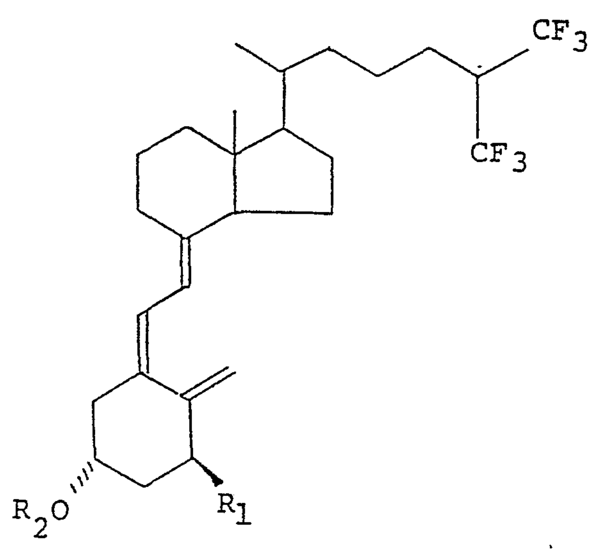

[IV]

wherein $R_1$ is a hydrogen atom, a hydroxyl group or a protected hydroxyl group and $R_2$ is a hydrogen atom or a

protecting group, which comprises subjecting a previtamin $D_3$ derivative of the formula [III]

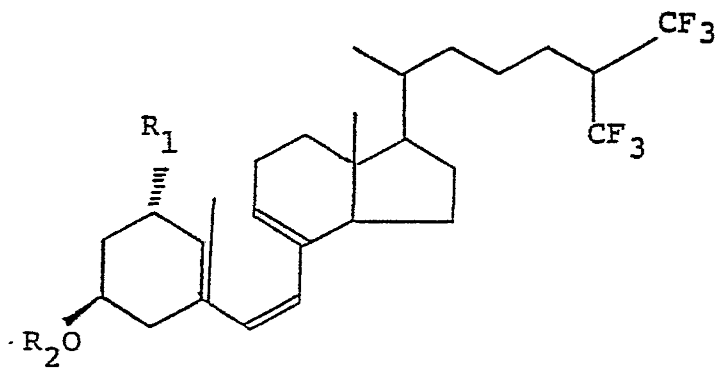

[III]

wherein $R_1$ and $R_2$ are as defined above, to thermal isomerization to yield a vitamin $D_3$ derivative of the formula [IV] mentioned above and, when $R_1$ is a protected hydroxyl group or when $R_2$ is a protecting group, subjecting, as required, the derivative obtained above to deprotection reaction to give a product of the formula [I]

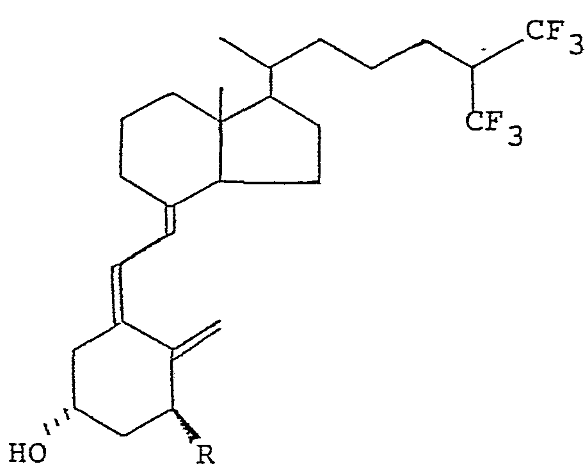

[I]

wherein R is a hydrogen atom or a hydroxyl group.

9. A compound of the formula [II]

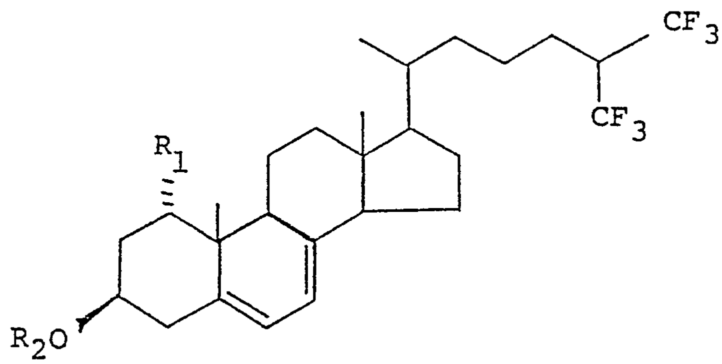

[II]

wherein $R_1$ is a hydrogen atom, a hydroxyl group or a protected hydroxyl group and $R_2$ is a hydrogen atom or a protecting group.

10. A compound of the formula [III]

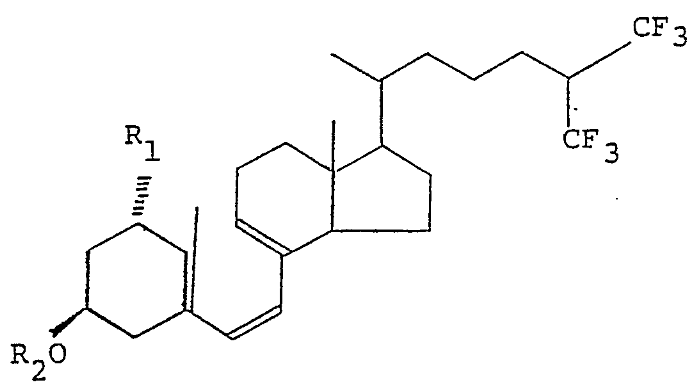

[III]

wherein $R_1$ is a hydrogen atom, a hydroxyl group or a protected hydroxyl group and $R_2$ is a hydrogen atom or a protecting group.

European Patent Office

# EUROPEAN SEARCH REPORT

0205025

Application number

EP 86 10 7131

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,Y | US-A-4 358 406 (HECTOR F. DELUCA)<br>* Claims 1,2,4,9; columns 1-4 *<br>--- | 1-10 | C 07 C 172/00<br>A 61 K 31/59<br>C 07 J 9/00 |
| D,Y | US-A-4 248 791 (HECTOR F. DELUCA)<br>* Claims; column 5-7 *<br>--- | 1-10 | |
| D,Y | PROCEEDING OF THE NATIONAL ACADEMY OF SCIENCES, vol. 80, January 1983, pages 201-204, Washington, D.C., US; Y. HONMA et al.: "1alpha,25-Dihydroxyvitamin D3 and 1alpha-hydroxyvitamin D3 prolong survival time of mice inoculated with myeloid leukemia cells"<br>* Whole document *<br>--- | 1,5-7 | TECHNICAL FIELDS SEARCHED (Int. Cl.4)<br>C 07 C 172/00 |
| Y | CHEMICAL ABSTRACTS, vol. 102, no. 1, 7th January 1985, page 549, abstract no. 6060r, Columbus, Ohio, US; JP - A - 59 116 283 (CHUGAI PHARMACEUTICAL CO. LTD.) 05-07-1984<br>* Abstract *<br>--- -/- | 1,5-7 | |

The present search report has been drawn up for all claims

| Place of search | Date of completion of the search | Examiner |
|---|---|---|
| THE HAGUE | 27-08-1986 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

0205025
Application number

EP 86 10 7131

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 103, no. 15, 14th October 1985, page 83, abstract no. 116282t, Columbus, Ohio, US; JP - A - 60 64 925 (SANKYO CO. LTD.) 13-04-1985 * Abstract * --- | 1,5-7 | |
| D,P X | CHEMICAL ABSTRACTS, vol. 104, no. 20, 19th May 1986, page 391, abstract no. 174664a, Columbus, Ohio, US; JP - A - 61 07 215 (TAISHO PHARMACEUTICAL CO. LTD.; SUMITOMO CHEMICAL CO. LTD.) 13-01-1986 * Abstract * ----- | 1,5-7 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 27-08-1986 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document